# EUROPEAN PATENT SPECIFICATION

(11) **EP 1 357 184 B1**
(45) Date of publication and mention of the grant of the patent: **30.10.2013**
(21) Application number: 03007054.4
(22) Date of filing: 27.03.2003
(51) Int. Cl.: C12N 15/11, C07H 21/00, A61K 31/00

(54) **Novel cis-element decoys useful as anti-tumor therapeutics**
Neue cis-Element-Fallen zur Verwendung als Anti-Tumor-Therapeutika
Nouveaux leurres d'elements cis utiles comme agents thérapeutiques antitumoraux

(30) Priority: 28.03.2002 CH 2112818
(43) Date of publication of application: 29.10.2003
(73) Proprietor: Nanjing Keygen Biotech. Co., Ltd., East Road Nanjing, (210002) P.R. China (CH)
(72) Inventor: Ye, Qing, 486 Zhongshan, East Rd. Nanjing (210002) (CH); Wang, Xuegen, 486 Zhongshan, East Rd. Nanjing (210002) (CH); Cheng, Wuling, 486 Zhongshan, East Rd. Nanjing (210002) (CH); Feng, Ying, 486 Zhongshan, East Rd. Nanjing (210002) (CH)
(74) Representative: TBK

(56) References cited:
- EP-A- 0 572 287
- WO-A-95/11687
- WO-A-99/26634
- US-A- 5 683 985
- US-A- 5 780 262
- US-A- 5 844 096
- LEE YOUL NAM ET AL: "CRE-transcription factor decoy oligonucleotide inhibition of MCF-7 breast cancer cells: Cross-talk with p53 signaling pathway" BIOCHEMISTRY, vol. 39, no. 16, 25 April 2000 (2000-04-25), pages 4863-4868, XP002264482
- ALPER OZGE ET AL: "Apoptosis, growth arrest and suppression of invasiveness by CRE-decoy oligonucleotide in ovarian cancer cells: Protein kinase A downregulation and cytoplasmic export of CRE-binding proteins" MOLECULAR AND CELLULAR BIOCHEMISTRY, vol. 218, no. 1-2, February 2001 (2001-02), pages 55-63, XP009022870
- KUME MASAZUMI ET AL: "Administration of a decoy against the activator protein-1 binding site suppresses neointimal thickening in rabbit balloon-injured arteries" CIRCULATION, vol. 105, no. 10, 12 March 2002 (2002-03-12), pages 1226-1232, XP002273848
- SWANSON HOLLIE I ET AL: "Specificity of DNA binding of the c-Myc/Max and ARNT/ARNT dimers at the CACGTG recognition site" NUCLEIC ACIDS RESEARCH, vol. 27, no. 15, 1 August 1999 (1999-08-01), pages 3205-3212, XP002273849

## Description

### FIELD OF THE INVENTION

The present invention relates to compositions of synthetic oligonucleotides as cis-element decoys for competitive binding of targeted transcription factors and their applications in the field of tumor biology and anti-tumor therapeutics.

### BACKGROUND OF THE INVENTION

One of the characteristics of tumor cells is the overexpression or amplification of proto-oncogenes. For example, C-MYC is found to be overexpressed in leukemia, lymphoma and sarcoma and is amplified to different extents in leukemia, breast cancer, stomach cancer, small cell lung cancer, colon cancer, neuroblastoma, and glioblastoma. Other examples include the overexpression of C-FOS in osteosarcoma and lymphoma and C-JUN is found to be highly overexpressed in small cell lung cancer, non-small cell lung cancer, colon and rectal cancers. In addition, C-MYC and C-JUN are found to be overexpressed at tumor's initiation and incubation stages (to be linked to the appearance of tumors). Finally, the amplification of C-MYC is one of the pre-requests for tumorigenesis.

Another characteristics of tumor cells is the loss of cell cycle control and the proper reaction to the cell growth factors. The heterodimer MYC/MAX binds to E-box to activate the expression of itself and its down stream genes such as CYC25A, leading to the formation of the cell growth phenotype. Together with E2F, the heterodimer can also activate cyclinA and cyclinE, a key event for the G1 to S phase transition in the cell cycle. C-MYC is found to activate the expression of telomerase and play an important role in tumor formation, while C-JUN and C-FOS are important in the down stream nucleus phase signal transduction of the RAS pathway. Furthermore, C-MYC, C-FOS and C-JUN are important in cell apoptosis, genome stability and cell maturation. Therefore, C-MYC, C-FOS and C-JUN can act as drug targets. By fixing the abnormal expression of these three proto-oncogenes, the cancerous hypergrowth phenotype can potentially be transformed into a benign and normal phenotype.

Proto-oncogenes and tumor suppressor genes coexist in normal and tumor cells. The difference between normal and tumor cells lies in the expression levels of the two types of genes. In the tumor cells, the proto-oncogenes are overexpressed by 2-10 times while the tumor suppressor genes are inactivated. These abnormalities are closely related to the binding status, type and quantity of the cistrons of the regulatory regions of said genes. Removing the abnormalies by changing the gene regulatory states is a novel approach for oncogene therapy.

Transcription factor decoys utilizing the specific DNA sequences for binding to transcription factors in order to change the gene expression levels, therefore leading to changes in cell phenotypes, can be developed into an effective tumor therapy.

Studies in DNA-protein interactions form the experimental basis for developing nucleic acid decoys as therapeutics. After a short piece of *in vitro* synthesized DNA with a specific sequence binds to certain protein factor extracted from the nucleus, the resulting DNA-protein complex band is shifted compared to the free DNA in polyacrylamide gel electrophoresis. Since Bielinska (1990) proposed the application of double-stranded oligonucleotides to regulate gene expression in the 90's, nucleic acid decoys have been initially used in cardiovascular drug development. For example, nucleic acid decoy therapy targeting the E2F factor for the treatment of inner membrane growth after CABG has entered clinical trials (Mann et al, 1999). Nucleic acid decoys targeting NF-kappa β for the treatment of ischemia was also reported (Sawa, 1997; Tounita, 1998). The application of nucleic acid decoys in tumor therapy is in its early stages. Nucleic acid decoys for the CREB factor (Cho-chong, 1999) has been tested in multiple tumor cell lines and in animal models. Nucleic acid decoys for ER has been used to inhibit breast cancer cell growth (Piva R, et. al, 200). Nuclei acid decoys for NF-kappa β has been found to inhibit mouse tumor growth both *in vitro* and *in vivo.*

WO 95 11687 discloses that decoy oligonucleotides can inhibit binding of transcription factors to DNA and thus inhibit the cell growth.

Dam et al., Oncogene 20: 2453-2464 discloses that oligonucleotides consisting of TTACCTCA sequence may bind to transcription factor JUN/ATF dimers that are associated with oncogenesis.

### SUMMARY OF THE INVENTION

The present invention covers oncogene switching therapeutics that are composed of sequence-specific nucleic acid decoys. These kinds of nucleic acids can competitively combine with certain oncogene transcription factors, and regulate the expression of the proto-oncogene C-JUN, leading to the inhibition of tumor cell growth and attaining the objective of tumor treatment.

The primary objective of the present invention is to develop pharmaceutical compositions comprising one or a combination of the nucleic acid decoys.

Another objective of the present invention is to apply said nucleic acid decoys as therapeutics for anti-cancer drugs.

These oncogene switching therapeutics use the specific sequences that bind to transcription factors as the core sequences of the nucleic acid decoys. In order to increase the drugs' stability and affinity for drug targets, the core sequences are expanded in both directions with more nucleotides so that the longer sequences can form three-dimensional structures.

The present invention discloses several pharmaceutical composition of nucleic acid decoys molecules specifically target the drug targets of transcription factors of the JUN/ATF transcription machinery. Said decoys are shown to inhibit tumor cell growth and possess great promises as anti-cancer drug candidates. The said pharmaceutical composition can be combined with carriers consisted of saline, buffer, liposome and polymers to be made into formulations consisted of injectables, oral capsules and tablets, bolus, suppository and skin applicants. Said pharmaceutical composition can also be used in combination with chemotherapeutics and radiation therapies to enhance their efficacies.

### BRIEF DESCRIPTION OF THE FIGURES

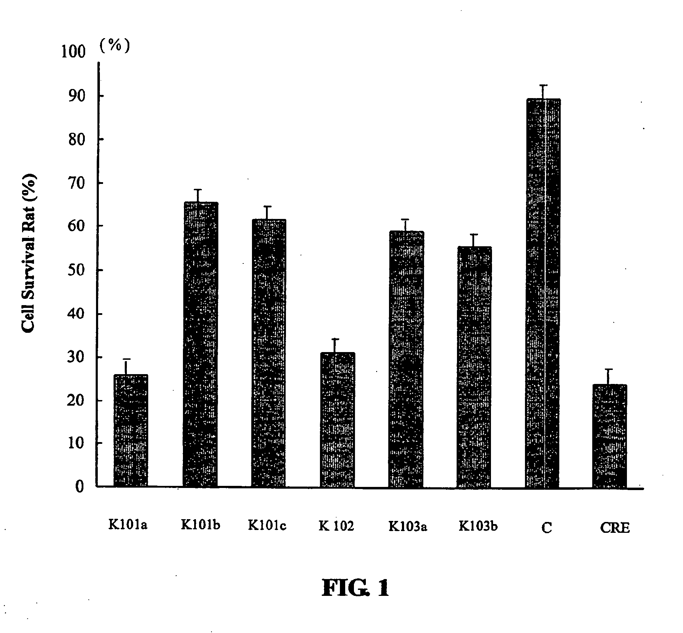

FIG. 1. Growth inhibition of NCI-H460 tumor cells by KGCD series nucleic acid decoys. Growth inhibition of NCI-H460 tumor cells by 6 KGCD series nucleic acid decoys is shown in FIG. 1. The cell survival rate is calculated by SRB staining and measuring the OD values, with blank control as 100%. CRE represents positive control, C is the negative control and the rest 6 groups are KGCD series decoys. Fig. 1 shows NCI-H460 cell growth inhibition to various degrees by the decoys, with K101a and K102 exhibiting the most severe growth inhibition.

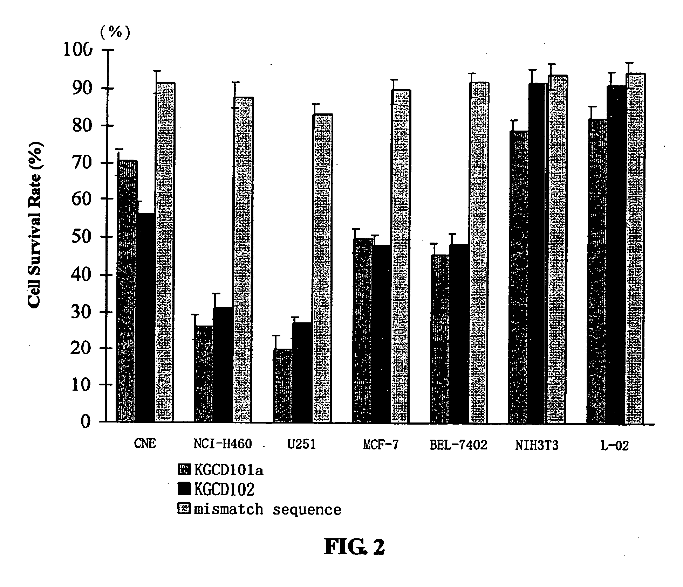

FIG. 2. Growth inhibition of various tumor cell lines by nucleic acid decoys K101a and K102. K101a and K102 are used to treat the following tumor cell lines: NCI-H460 (lung cancer), CNE (head and neck cancer), U251 (neuroglioblastoma), MCF-7 (breast cancer), BEL-7402 (liver cancer) and normal cell lines L-02 (liver cell) and NIH3T3. The column with the faintest color represents negative control (scrambled sequence), showing little influence on cell growth. K101a and K102 inhibit the growth of all the tumor cell lines to different degrees with the growth inhibition to U251 and NCI-H460 the most severe. The decoys have little effective on the growth of normal cells.

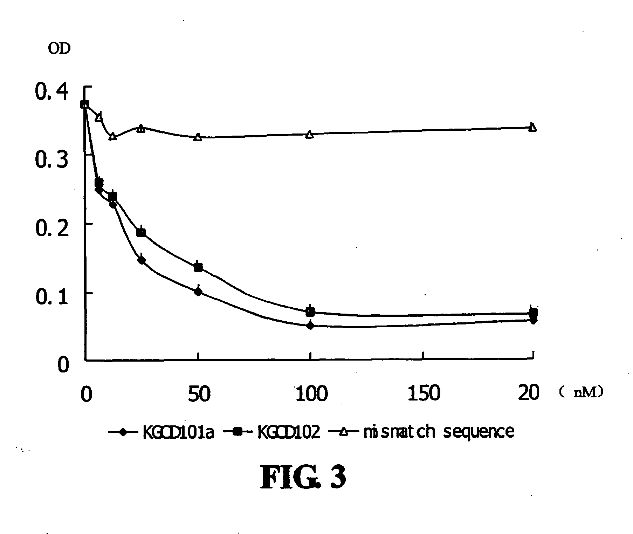

FIG. 3. IC₅₀ of NCI-H460 tumor cells by nucleic acid decoys K101a and K102. Tumor cell line NCI-H460 is treated with different concentrations of K101a and K102. As shown in Fig. 3, K101a and K102 can inhibit the growth of NCI-H460 at relatively low concentrations with IC₅₀ around 30-50 nM. The negative control group shows no dose dependent effect on cell growth.

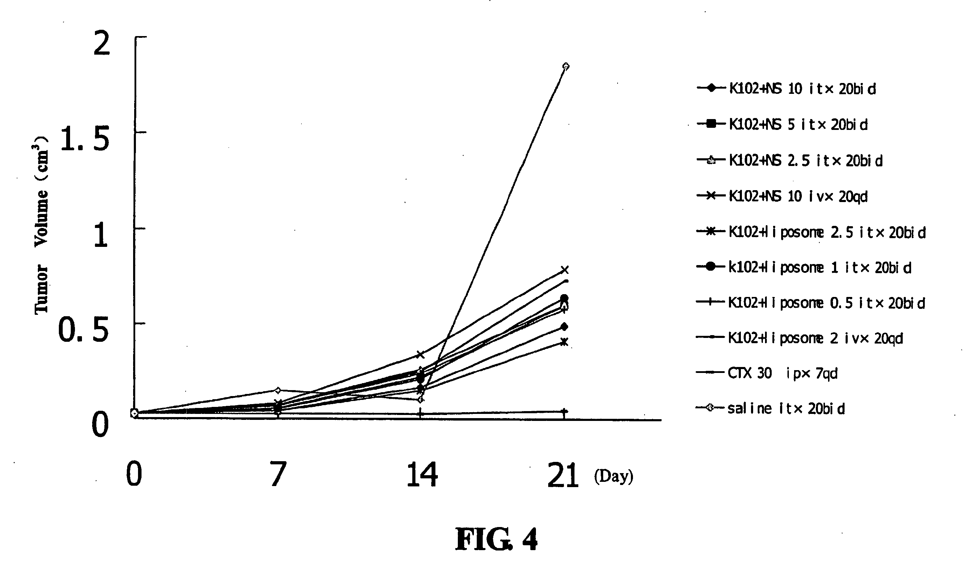

FIG. 4. Tumor volume changes of nude mice inoculated with human lung cancer cell line NCI-H460 after treatment by AK102. Groups of decoy drugs with different concentrations and formulations exhibit tumor growth inhibition to difference degrees 7 days after drug administration. The liposome group with 2.5 mg/kg concentration shows the highest growth inhibition of 77% (V/V) after 20 days of drug administration.

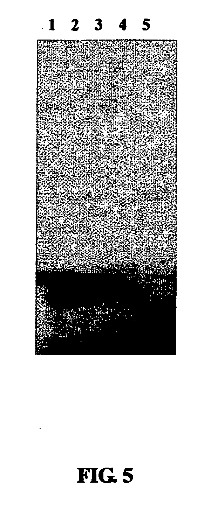

FIG. 5. Gel shift assay with K102 in polyacrylamide gel electrophoresis. Panel 1, 3: Complex formed between ³²P-K102 and cell nucleus extracts, Panel 2, 4: Complex formed between ³²P-K102 and cell nucleus extracts in the presence of unlabeled K102 (as specific competitor), Panel 5: ³²P-K102 only, no cell nucleus extracts. Results indicate K102 formed specific complex with nuclear proteins.

### DETAILED DESCRIPTION OF THE INVENTION

The therapeutic K101A series (outside the scope of the invention) are designed to regulate the overexpression of the proto-oncogene C-FOS. Since the transcription factor CREB binds to the proto-oncogene C-FOS' enhancer CRE sequence STGACGTMR (seq. 1, SEQ ID NO: 1), seq.1 can be used as the core sequence of nucleic acid decoys to competitively bind to CREB and regulate the expression levels of the proto-oncogene C-FOS. For example, K101a (seq. 6, SEQ ID NO: 6) is a single-stranded 23-mer oligonucleotide that forms a hairpin structure. K101a-1 is composed of a cruciform through the binding of seq. 7 (SEQ ID NO:7) and seq. 8 (SEQ ID NO: 8) that contain said core sequence. K101a-2 is a double stranded linear structure formed by seq. 9 (SEQ ID NO: 9) that contains the core sequence. In summary, the therapeutic K101A series are single or double stranded DNA molecules 15-40 nucleotides long that contain the core sequence STGACGTMR.

The therapeutics K101b and K101c (outside the scope of the invention) are designed to regulate the expression of multiple proto-oncogenes. The AP-1 transcription factor is a heterodimer of JUN/FOS that binds to the sequence STGASTMA (seq. 2, SEQ ID NO: 2) that is a cis-element to multiple genes and activates the expression of these genes. Said sequence is used as the core sequence in the nucleic acid decoy which can down-regulate the expression levels of the genes that contain the AP-1 enhancer. For example, K101b is a double stranded DNA molecule that is formed by seq. 10 (SEQ ID NO: 10) that are 20-mer oligonucleotides containing said core sequence. K101b-1 (seq. 11, SEQ ID NO: 11) is a hairpin structure formed by a single stranded 21-mer oligonucleotide containing said core sequence. K101b-2 (seq. 12, SEQ ID NO: 12) is a 42-mer oligonucleotide that contains said core sequence. A ligase is used to form a closed loop by link K101b-2's 5' end with its 3' end. The resulting dumbbell structure enhances the drug's stability. K101c1 (seq. 13, SEQ ID NO: 13) and K101c2 (seq. 14, SEQ ID NO: 14) is comprised of 22-mer and 21-mer single-stranded oligonucleotides, respectively; K101c3 (seq. 15, SEQ ID NO: 15) and K101c4 (seq. 16, SEQ ID NO: 16) is comprised of 23-mer and 21-mer single-stranded oligonucleotides, respectively. All four therapeutics form the hairpin structure or become double stranded through self annealing and all contain said core sequence. In summary, the therapeutics K101b and K101c series are single or double stranded DNA molecules 15-40 nucleotides long that contain the sequence STGASTMA.

In one preferred embodiment, the therapeutic K102 series are design to use TTACCTCA (seq. 3, SEQ ID NO: 3) as the core sequence of the nucleic acid decoy to regulate the expression of the proto-oncogenes such as C-JUN. Said sequence is the conserved enhancer sequence that C-JUN binds to enhance its own expression. At the same time, the JUN/ATF protein heterodimer as a transcription factor also binds to said sequence to regulate the expression of the genes that have said sequence as an enhancer. For instance, K102 (seq. 17, SEQ ID NO: 17) is a single stranded 23-mer oligonucleotide hairpin structure that contain said core sequence. K102-1 (seq. 18, SEQ ID NO: 18) is a 21-mer oligonucleotide hairpin structure, K102-2 (seq. 19, SEQ ID NO: 19) is a 22-mer oligonucleotide, K102-3 (seq. 20, SEQ ID NO: 20) is a single stranded 20-mer oligonucleotide and K102-4 (seq. 21, SEQ ID NO: 21) is a linear double-stranded 20-mer nucleotide. In summary, the therapeutic K102 series are single or double stranded DNA molecules 15-40 nucleotides long that contain the sequence TTACCTCA.

The therapeutic K103a (outside the scope of the invention) is designed to regulate the expression of proto-oncogene C-MYC. Since C-MYC binds to its own enhancer TCTCTTA (seq. 4, SEQ ID NO: 4), nucleic acid decoy drugs are designed to incorporate said core sequence to down regulate the expression levels of C-MYC. For example, K103a is a double stranded DNA comprised of seq. 22 (SEQ ID NO: 22), both of which contain said core sequence. K103a-1 (seq. 23, SEQ ID NO: 23) is a 25-mer oligonucleotide hairpin structure that contains said core sequence. 103a-2 is a 43-mer oligonucleotide stem-loop structure comprised of seq. 24 (SEQ ID NO: 24) and seq. 25 (SEQ ID NO: 25), both of which contain said core sequence. K103a-3 is a 36-mer oligonucleotide hammer structure comprised of seq. 26 (SEQ ID NO: 26) and seq. 27 (SEQ ID NO: 27) both of which contain said core sequence. In summary, the therapeutic K103a series are single or double stranded DNA molecules 15-40 nucleotides long that contain the sequence TCTCTTA.

The therapeutic K103b (outside the scope of the invention) is designed to regulate the expression levels of genes associated with the cell cycle regulation. The protein heterodimer MYC/MAX, through binding to the cis-element RACCACGTGGTY (seq. 5, SEQ ID NO: 5), causes cell proliferation and the loss of cell cycle control. Nucleic acid decoy drugs are designed by incorporating said sequence as the core sequence. After they are synthesized *in vitro* and introduced into the cells, said drugs compete with the said cis-element for the binding of MYC/MAX heterodimer, leading to the repression of the down stream gene expression and suppression of the malignant growth phenotype. For example, K103b (seq. 28, SEQ ID NO: 28) is a 24-mer oligonucleotide containing said core sequence. K103b-1 (seq. 29) and K103b-1 (seq. 30) are 24-mer single-stranded oligonucleodes that can self-anneal to form double-stranded dimer and that also contain said core sequence. In summary, the therapeutic K103b series are single or double stranded DNA molecules 15-40 nucleotides long that contain the sequence RACCACGTGGTY.

The nucleic acid decoy drugs are synthesized by automatic DNA synthesizer. After phosphorothioate, de-protection, purification and lyophilization, they are dissolved in water for qualitative and quantitative analysis. NCI-H460 lung cancer cell lines are used for *in vitro* functional screening and efficacy testing. The said cells are added to 96-well Petri dishes and incubated for 24 hours with the complete culture medium RPMI 1640. The nucleic acid decoy drug mixed with liposome is added after the incubation and the cells are further incubated at 37°C, 5% CO₂ for another 48 hours. The cell survival rate is determined by SRB colorimetry. The screening results indicate that all six series of nucleic acid decoy drugs can inhibit the growth of NCI-H460 to different degrees with K101a and K102 showing the most severe inhibition. All these nucleic acid decoys can be developed into anti-cancer therapeutic drugs.

The pharmaceutical composition claimed above acts on JUN/ATF as novel targets of tumor therapies. Said protein molecules are valuable druggable targets due to their involvement in the regulation of the expression levels of multiple genes and are highly correlated with tumorigenesis.

Among the many potential methods to develop therapeutics against said drug targets, the nucleic acid transcription regulation decoys employed in this invention possess the advantages of a novel mechanism of action, clearly defined and specific functional targets with specific drug sequences. Said decoy drugs have been shown to effectively inhibit tumor cell growth in *in vitro* experiments. These nucleic acid decoys are much smaller molecules that can be easily formulated into deliverable drugs when compared to gene therapy tumor treatments. Its advantage over anti-sense drugs lies in its double-stranded structure, which is more stable *in vivo* than single stranded anti-sense drugs. In addition, decoy drugs require less quantity for efficacy and act on totally new targets. As a result, said decoy drugs qualify as novel therapeutic entities.

The core sequences of said nucleic acid decoy drugs are 6-12 nucleotides long. These short sequences are easily degraded. The double stranded structures they form are not stable and easily denatured in room temperatures. In addition, the lack of two-dimensional structure makes them less likely to bind to proteins. To overcome these shortcomings, decoy drugs are improved by adding more nucleotides to elongate the chains for the purpose of increased stability and affinity to transcription factors. Considering the hairpin, cruciform, stem and loop and dumbbell structures are the favorable secondary structures, said nucleic acid decoy drugs are designed to contain the said core sequences with total length of 15-55 nucleotides. The resulting sequences can stay as single stranded, self anneal or hybridize to form various secondary structures. To avoid degradation by ribonucleases *in vivo* and enhance stability, the decoy drug sequences are chemically modified, and phosphorothioate is the most common protection method. In addition, in order to increase decoy molecules' membrane penetration and bioavailability, lipophilic groups, such as cholesterol are attached to either or both ends of the decoy molecules. The liposome method can also be used for the same purpose. Furthermore, targeting molecules, such as antibodies can also be attached to increase efficacy by concentrating the decoys preferentially in the tumors.

In conclusion, pharmaceutical composition of nucleic acid decoy molecules with, JUN/ATF as the drug targets are described. Said decoys are shown to inhibit tumor cell growth and possess great promises as anti-cancer drug candidates. The said pharmaceutical composition can be combined with carriers consisted of saline, buffer, liposome and polymers to be made into formulations consisted of injectables, oral capsules and tablets, bolus, suppository and skin applicants. Said pharmaceutical composition can also be used in combination with chemotherapeutics and radiation therapies to enhance their efficacies.

### EXAMPLES

The following examples are intended to illustrate, but not limit, the scope of the invention.

### REFERENCE EXAMPLE 1

### Growth inhibition of lung cancer cell line NCI-H460 by 6 KGCD series nucleic acid decoys

Six phosphorothioate nucleic acid decoys as shown below are synthesized: K101a (seq. 6), K101b (seq. 10 and seq. 11), K101c1 (seq. 14), K102 (seq. 18), K103 (seq. 23 and seq. 24) and K103b (seq. 30).
Negative control sequence: 5'—TGTGGTCATGTGGTCATGTGTCA—3'
Positive control sequence: 5'—TGACGTCATGACGTCATGACGTCA—3'

The lung cancer cell line NCI-H460 is divided into positive control, negative control, blank control and the six KGCD series nucleic acid decoy experimental groups. The cells are transferred into a 96 well Petri dish containing RPMI 1640 with 10% fetal bovine serum at 2-3 X 10⁴/well. After incubating for 24 hours, the culture medium is changed to RPMI 1640 alone. 0.6 µl/well liposome and a final concentration of 200 nM of the various nucleic acid decoys are added (for blank control, only 0.6 µl/well liposome is added). After 5 hours, the medium is changed to RPMI 1640 with 10% fetal bovine serum. The cells are further incubated 37°C and 5% CO2. After 48 hours, the cells are stained with SRB and the cell survival rates are determined by the OD values measured at λ₅₁₀nm with a colorimeter. A cell growth inhibition graph is plotted with the blank control as 100%. Results are shown in Fig. 1: NCI-H460 cell growth is inhibited to various degrees by the decoys, with K101a and K102 exhibiting the most severe growth inhibition.

### REFERENCE EXAMPLE 2 AND EXAMPLE 1

### Growth inhibition of various tumor cell lines by nucleic acid decoy K101a (Reference Example; outside the invention) and K102.

The nucleic acid decoys are incubated with the following tumor cell lines: NCI-H460 (lung cancer), CNE (head and neck cancer), U251 (neuroglioblastoma cell), MCF-7 (breast cancer), BEL-7402 (liver cancer) and normal cell lines L-02 (liver cell) and NIH3T3. The column with the faintest color represents the negative control (scrambled sequence), showing little influence on cell growth. K101a and K102 inhibit the growth of all the tumor cell lines to different degrees with the growth inhibition to U251 and NCI-H460 the most severe. The decoys have little effective on the growth of normal cells.

### REFERENCE EXAMPLE 3 AND EXAMPLE 2

### ICso of NCI-H460 tumor cells by nucleic acid decoy K101a (Reference Example; outside the invention) and K102.

Tumor cell line NCI-H460 is treated with different concentrations of K101a and K102. After certain periods of incubation, OD values are measured to determine the cell survival rates. As shown in Fig. 3, K101a and K102 can inhibit the growth of NCI-H460 at relatively low concentrations with IC₅₀ around 30-50 nM, or 30-50 µg/ml, far higher than the anti-cancer drug screening standard (IC50<10 µg/ml).

### EXAMPLE 3

### Anti-cancer efficacy study of the therapeutic K102 in animals

The therapeutic K102 is formulated with saline and liposome. 24 hours after T-cell deficient nude mice were inoculated with the tumor cell line NCI-H460 subcutaneously, K102 is administered daily into the tumor or i.v. at these doses: 10 mg/kg, 5 mg/kg and 2 mg/kg for the saline formulation and 2.5 mg/kg, 1 mg/kg and 0.5 mg/kg for the liposome formulation. Saline and pure liposome are used as controls. The tumor volume is measured dynamically. After 20 days of continuous drug administration, the animals are euthanized and the tumors are dissected and weighed. In the saline formulation group with it X 20 bid, tumor growth is inhibited by 54.1%, 50.94% and 49.06% (W/W), respectively while the i.v. X 20 qd group achieved tumor inhibition of 39.06%. For the liposome formulation group with it X 20 bid, tumor growth is inhibited by 55.04%, 49.86% and 43.88% while the i.v. X 20 qd group achieved 43.74% tumor inhibition. Fig. 4 shows the tumor volume dynamic changes of nude mice inoculated with human lung cancer cell line NCI-H460 after treatment with AK102. It demonstrated that K102 is an effective inhibitor of tumor growth, independent of formulation.

### EXAMPLE 4

### Gel shift assay

Nuclear extracts are prepared by the method of Digna et al. Oligonucleotides are ³²P-labeled with [γ³²P]ATP, using polynucleotide kinase. Binding reaction mixture containing 0.5 ng probe, 5 µg nuclear extract, 2 µg of poly (dI-dC) (to inhibit non-specific binding of K102 with nuclear proteins) and binding buffer [10 mM Tris (pH 7.5), 50 mM NaCl, 1 mM DTTI, 1 mM EDTA, 5% glycerol] is incubated for 20 minutes at the room temperature. The DNA-protein complexes are resolved by electrophoresis through 4% poly-acrylamide gels containing 50 mM Tris, 0.38 M glycine and 2 mM EDTA. The gels are subsequently dried and autoradiographed with intensifying screens at -70°C. Results shown in Fig. 5 indicate that K102 forms specific complex with nuclear proteins, providing the support for the mechanism of action for K102.

This invention covers anti-cancer or oncogene switching therapeutics that employ sequence-specific nucleic acid decoys for competitive binding of transcription factors. These nucleic acid decoys can competitively bind to transcription factors regulating the expression of oncogenes including JUN/ATF in the cell, leading to the inhibition of tumor cell growth. As a result, they can be developed into anti-cancer therapeutic drugs.

## Claims

1. A composition comprising a polynucleotide with a length of 15-55 nucleotides comprising the sequence 5'- TTACCTCA -3' having a double-stranded structure and having affinity for binding to transcription factor JUN/ATF in a host cell,
wherein said polynucleotide sequence comprises a sequence selected from the group consisting of SEQ ID NO: 17 to 21.

2. The composition of claim 1, wherein said host cell is a mammalian cell.

3. The composition of claim 2, wherein said mammalian cell is a human cell.

4. The composition of claim 3, wherein said human cell is a human cancer cell.

5. The composition of claim 1, wherein said polynucleotide can be either double-stranded or single-stranded, wherein the said single-stranded structure can, through intra-chain or inter-chain interaction, form double-stranded or secondary structures consisting of hairpin, pseudo-knot, stem and loop, dumb-bell or cruciform.

6. The composition of claim 1, wherein the said polynucleotide comprises a nucleotide analog.

7. The composition of Claim 6, wherein said nucleotide analogs are selected from the group consisting of deoxyuracil, labeled nucleotide, ribonucleotide, 7-deaza-dNTP, methylthio-linked nucleotide, phosphothio-linked nucleotide, morpholino nucleotide, hexose-containing nucleotide, peptide nucleic acid (PNA) and their derivatives.

8. The composition of claim 6, wherein said labeled nucleotide is attached with a ligand molecule selected from the group consisting of lipophilic substrate, target-specific antibodies or other molecules with targeting functions and a combination thereof.

9. The composition of claim 1, wherein said polynucleotide is formulated into pharmaceutical formulation selected from the groups consisting of injectable, oral, transdermal, bolus, aerosol, suppository, formulations and the combination thereof.

10. A composition according to claim 1 comprising a polynucleotide with a length of 15-40nucleotides.

11. A method for regulating gene transcription and cell growth in a target cell comprising:
a) providing one or more transcription regulatory element decoys comprising a polynucleotide, wherein said polynucleotide is defined according to claim 1;
b) exposing said target cell to said polynucleotide under conditions such that said polynucleotide alters gene transcription and cell growth,
wherein the method is not practised on the human or animal body.

12. The method of claim 11, wherein the said target cell is a human cancer cell.

13. The method of claim 11, wherein said exposing is selected from the group consisting of injection, direct exposure, transfection, and transgenic expression not being practised on the human or animal body.

## Patentansprüche

1. Zusammensetzung, umfassend ein Polynucleotid mit einer Länge von 15-55 Nukleotiden, umfassend die Sequenz 5'-TTACCTCA-3' mit einer doppelsträngigen Struktur und mit Affinität zum Binden an den Transkriptionsfaktor JUN/ATF in einer Wirtszelle,
wobei die Polynucleotidsequenz eine Sequenz umfasst ausgewählt aus der Gruppe bestehend aus SEQ ID NR: 17 bis 21.

2. Zusammensetzung nach Anspruch 1, wobei die Wirtszelle eine Säugetierzelle ist.

3. Zusammensetzung nach Anspruch 2, wobei die Säugetierzelle eine menschliche Zelle ist.

4. Zusammensetzung nach Anspruch 3, wobei die menschliche Zelle eine menschliche Krebszelle ist.

5. Zusammensetzung nach Anspruch 1, wobei das Polynucleotid entweder doppelsträngig oder einzelsträngig sein kann, wobei die einzelsträngige Struktur durch Intra-Ketten- oder Inter-Ketten-Interaktion doppelsträngige oder sekundäre Strukturen bilden kann, die aus Haarnadel, Pseudoknoten, Stamm und Schlaufe, Hantel oder Kreuzform besteht.

6. Zusammensetzung nach Anspruch 1, wobei das Polynucleotid ein Nukleotidanaloga umfasst.

7. Zusammensetzung nach Anspruch 6, wobei die Nukleotidanaloga ausgewählt sind aus der Gruppe bestehend aus Deoxyuracil, gelabelten Nukleotid, Ribonucleotid, 7-Deaza-dNTP, Methylthio-gebundenem Nukleotid, Phosphothio-gebundenem Nukleotid, Morpholinonucleotid, Hexose-enthaltendem Nukleotid, Peptidnucleinsäure (PNA) und deren Derivate.

8. Zusammensetzung nach Anspruch 6, wobei das gelabelte Nukleotid mit einem Ligandmolekül angefügt ist, das aus der Gruppe ausgewählt ist, die aus lipophilem Substrat, Ziel-spezifischen Antikörpern oder anderen Molekülen mit zielenden Funktionen und einer Kombination davon besteht.

9. Zusammensetzung nach Anspruch 1, wobei das Polynucleotid in eine pharmazeutische Formulierung formuliert ist, ausgewählt aus den Gruppen bestehend aus injizierbar, oral, transdermal, Pille, Aerosol, Zäpfen, Formulierungen und Kombinationen davon.

10. Zusammensetzung nach Anspruch 1, umfassend ein Polynucleotid mit einer Länge von 15-40 Nukleotiden.

11. Verfahren zur Regulierung der Gentranskription und des Zellwachstums in einer Zielzelle, umfassend:
a) Bereitstellen einer oder mehrerer transkriptionsregulatorischen Elementköder, umfassend ein Polynucleotid, wobei das Polynucleotid gemäß Anspruch 1 definiert ist;
b) Exponieren der Zielzelle zu dem Polynucleotid unter Bedingungen, so dass das Polynucleotid die Gentranskription und das Zellwachstum verändert, wobei das Verfahren nicht an dem menschlichen oder tierischen Körper praktiziert wird.

12. Verfahren nach Anspruch 11, wobei die Zielzelle eine menschliche Krebszelle ist.

13. Verfahren nach Anspruch 11, wobei das Exponieren ausgewählt ist aus der Gruppe bestehend aus Injektion, direkter Exponierung, Transfektion und transgenetischer Expression, die nicht an dem menschlichen oder tierischen Körper praktiziert werden.

## Revendications

1. Composition comprenant un polynucléotide ayant une longueur de 15 à 55 nucléotides qui comprend la séquence 5'- TTACCTCA -3' ayant une structure double brin et ayant une affinité pour se lier à un facteur de transcription JUN/ATF dans une cellule hôte,
où ladite séquence polynucléotidique comprend une séquence choisie dans le groupe constitué de SEQ ID NO: 17 à 21.

2. Composition de la revendication 1, dans laquelle ladite cellule hôte est une cellule de mammifère.

3. Composition de la revendication 2, dans laquelle ladite cellule de mammifère est une cellule humaine.

4. Composition de la revendication 3, dans laquelle ladite cellule humaine est une cellule cancéreuse humaine.

5. Composition de la revendication 1, dans laquelle ledit polynucléotide peut être double brin ou simple brin, où ladite structure simple brin peut, à travers une interaction intra-chaîne ou inter-chaîne, former des structures secondaires ou double brin constituées d'épingle à cheveux, de pseudo-noeud, de tige et boucle, d'haltère ou de cruciforme.

6. Composition de la revendication 1, dans laquelle ledit polynucléotide comprend un analogue de nucléotide.

7. Composition de la revendication 6, dans laquelle lesdits analogues de nucléotide sont choisis dans le groupe constitué de désoxyuracile, de nucléotide marqué, de ribonucléotide, de 7-déaza-dNTP, de nucléotide lié à un méthylthio, de nucléotide lié à un phosphothio, de nucléotide morpholino, de nucléotide contenant de l'hexose, d'acide nucléique peptidique (PNA) et de leurs dérivés.

8. Composition de la revendication 6, dans laquelle ledit nucléotide marqué est fixé à une molécule de ligand choisie dans le groupe constitué de substrat lipophile, d'anticorps spécifiques à la cible ou d'autres molécules ayant des fonctions de ciblage et d'une combinaison de ceux-ci.

9. Composition de la revendication 1, dans laquelle ledit polynucléotide est formulé en une formulation pharmaceutique choisie dans les groupes constitués de formulation injectable, orale, transdermique, de bolus, d'aérosol, de suppositoire, de formulations et de la combinaison de celles-ci.

10. Composition selon la revendication 1 comprenant un polynucléotide ayant une longueur de 15 à 40 nucléotides.

11. Procédé de régulation de la transcription génique et de la croissance cellulaire dans une cellule cible comprenant le fait :
a) de fournir un ou plusieurs leurres d'élément de régulation de la transcription comprenant un polynucléotide, où ledit polynucléotide est défini selon la revendication 1 ;
b) d'exposer ladite cellule cible audit polynucléotide dans des conditions telles que ledit polynucléotide modifie la transcription génique et la croissance cellulaire,
où le procédé n'est pas pratiqué sur le corps humain ou animal.

12. Procédé de la revendication 11, dans lequel ladite cellule cible est une cellule cancéreuse humaine.

13. Procédé de la revendication 11, dans lequel ladite exposition est choisie dans le groupe constitué d'une injection, d'une exposition directe, d'une transfection, et d'une expression transgénique qui n'est pas pratiquée sur le corps humain ou animal.


### SEQUENCE LISTING

(1) GENERAL INFORMATION:
   (iii) NUMBER OF SEQUENCES: 30
(2) INFORMATION FOR SEQ ID NO: 1:
   (i) SEQUENCE CHARACTERISTICS:
      (A) LENGTH: 9 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION: SEQ ID NO:1:
      STGACGTMR 9
(2) INFORMATION FOR SEQ ID NO:2:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: other nucleic acid
      (A)DESCRIPTION:/desc-# ="DNA"
   (xi) SEQUENCE DESCRIPTION: SEQ ID NO:2:
      STGASTMA 8
(2) INFORMATION FOR SEQ ID NO:3
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:3:
      TTACCTCA 8
(2) INFORMATION FOR SEQ ID NO:4
   (i) SEQUENCE CHARACTERISTICS:
      (A) LENGTH: 7 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:4:
      TCTCTTA 7
(2) INFORMATION FOR SEQ ID NO:5
   (i) SEQUENCE CHARACTERISTICS:
      (A) LENGTH: 12 base pairs
      (B) TYPE: nucleic acid
      (C) STRANDEDNESS:single
      (D) TOPOLOGY:linear
   (ii)MOLECULE TYPE: other nucleic acid
      (A)DESCRIPTION:/desc-# ="DNA"
   (xi)SEQUENCE DESCRIPTION:SEQ ID NO:5:
      RACCACGTGGTY 12
(2) INFORMATION FOR SEQ ID NO:6
   (i) SEQUENCE CHARACTERISTICS:
      (A) LENGTH: 23 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION: SEQ ID NO:6:
      GCTGACGTCA GACTGACGTC AGC 23
(2)INFORMATION FOR SEQ ID NO:7
   (i) SEQUENCE CHARACTERISTICS:
      (A) LENGTH: 24 base pairs
      (B) TYPE: nucleic acid
      (C) STRANDEDNESS:double
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:7:
      GTTTCGGGGT GACGTCACCC TTTC 24
(2) INFORMATION FOR SEQ ID NO:8
   (i) SEQUENCE CHARACTERISTICS:
      (A) LENGTH: 24 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:8:
      GAAAGGGACG TACGTCCGCG AAAC 24
(2) INFORMATION FOR SEQ ID NO:9
   (i) SEQUENCE CHARACTERISTICS:
      (A) LENGTH: 23 base pairs
      (B) TYPE: nucleic acid
      (C) STRANDEDNESS:double
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:9:
      AGATTGCCTG ACGTCAGAGA GCT 23
(2) INFORMATION FOR SEQ ID NO: 10
   (i) SEQUENCE CHARACTERISTICS:
      (A) LENGTH: 20 base pairs
      (B) TYPE: nucleic acid
      (C) STRANDEDNESS: double
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO: 10:
      CGCTTGCTGA CTCAGCCGGA 20
(2) INFORMATION FOR SEQ ID NO:11
   (i) SEQUENCE CHARACTERISTICS:
      (A) LENGTH: 21 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# "DNA"
   (xi) SEQUENCE DESCRIPTION:SEQ ID NO:11:
      TCAGTACTGA CTCAGTACTG A 21
(2) INFORMATION FOR SEQ ID NO:12
   (i) SEQUENCE CHARACTERISTICS:
      (A) LENGTH: 42 base pairs
      (B) TYPE: nucleic acid
      (C) STRANDEDNESS: single
      (D) TOPOLOGY: circular
   (ii) MOLECULE TYPE: other nucleic acid
      (A) DESCRIPTION:/desc-# ="DNA"
   (xi) SEQUENCE DESCRIPTION:SEQ ID NO:12:
      ACTCTCTCAG TCTGACTCAT GCTCTCAGCA TGAGTCAGAC TG 42
(2) INFORMATION FOR SEQ ID NO: 13
   (i) SEQUENCE CHARACTERISTICS:
      (A) LENGTH: 22 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO: 13:
      TCACGGTATG ACTCATCCGT GA 22
(2) INFORMATION FOR SEQ ID NO:14
   (i) SEQUENCE CHARACTERISTICS:
      (A) LENGTH: 21 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO: 14
      CACGGTCTGA CTCAGACCGT G 21
(2) INFORMATION FOR SEQ ID NO: 15
   (i) SEQUENCE CHARACTERISTICS:
      (A) LENGTH: 23 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:15
      TGAGTCAGTG ACTCACTGAC TCA 23
(2) INFORMATION FOR SEQ ID NO:16
   (i) SEQUENCE CHARACTERISTICS:
      (A) LENGTH: 21 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO: 16
      CTCCGGCTGA CTCAGCCGGA G 21
(2) INFORMATION FOR SEQ ID NO: 17
   (i) SEQUENCE CHARACTERISTICS:
      (A) LENGTH: 21 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTTON:SEQ ID NO:17
      GTTACCTCAG CCCGTGAGGT AAC 23
(2) INFORMATION FOR SEQ ID NO: 18
   (i) SEQUENCE CHARACTERISTICS:
      (A) LENGTH: 21 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:18
      GTTACCTCAG CCTGAGGTAA C 21
(2) INFORMATION FOR SEQ ID NO: 19
   (i) SEQUENCE CHARACTERISTICS:
      (A) LENGTH: 22 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:19
      GTTACCTCAC GCGTGAGGTA AC 22
(2) INFORMATION FOR SEQ ID NO:20
   (i) SEQUENCE CHARACTERISTICS:
      (A) LENGTH: 20 base pairs
      (B) TYPE: nucleic acid
      (C) STRANDEDNESS:single
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:20
      CGTTACCTCA TGAGGTAACG 20
(2) INFORMATION FOR SEQ ID NO:21
   (i) SEQUENCE CHARACTERISTICS:
      (A) LENGTH: 20 base pairs
      (B) TYPE: nucleic acid
      (C) STRANDEDNESS: double
      (D) TOPOLOGY:linear
   (ii) MOLECULE TYPE: other nucleic acid
      (A) DESCRIPTION:/desc-# ="DNA"
   (xi) SEQUENCE DESCRIPTION:SEQ ID NO:21
      ATTGCCTTAC CTCAGAGAGC 20
(2) INFORMATION FOR SEQ ID NO:22
   (i) SEQUENCE CHARACTERISTICS:
      (A) LENGTH: 26 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:22
      TCACCATCTC TTATGCGGTT GAATAG 26
(2) INFORMATION FOR SEQ ID NO:23
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:23
      TATGACTAAT CTCTRATTAG TCATA 25
(2) INFORMATION FOR SEQ ID NO:24
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:24
      TCTCTTAGCT CTCTTAGCCT CTCTTA 26
(2) INFORMATION FOR SEQ ID NO:25
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:25
      TAAGAGAGGC TAAGAGA 17
(2) INFORMATION FOR SEQ ID NO:26
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/desc-# ="DNA"
   (xi) SEQUENCE DESCRIPTION:SEQ ID NO:26
      GAGTCTCTTA CTCCCGCGG 19
(2) INFORMATION FOR SEQ ID NO:27
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/DESC-# ="DNA"
   (xi) SEQUENCE DESCRIPTION:SEQ ID NO:27
      CCGCGGGTCT CTTAGAC 17
(2) INFORMATION FOR SEQ ID NO:28
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/DESC-# ="DNA"
   (xi) SEQUENCE DESCRIPTION:SEQ ID NO:28
      CACGGAGACC ACGTGGTCTC CGTG 24
(2) INFORMATION FOR SEQ ID NO:29
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/DESC-# ="DNA"
   (xi) SEQUENCE DESCRIPTION:SEQ ID NO:29
      GAAGCAGAC CACGTGGTCT GCTTC 24
(2) INFORMATION FOR SEQ ID NO:30
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION:/DESC-# ="DNA"
   (xi) SEQUENCE DESCRIPTION:SEQ ID NO:30
      AACCACGTGG TTAACCACGT GGTT 24

### SEQUENCE LISTING

<110> Nanjing Keygen Biotech. Co., LTD
<120> Novel Cis-Element Decoys Useful as Anti-Tumor Therapeutics
<150> CN02112818.9
<151> 2002-03-28
<160> 30
<210> 1
   <211> 9
   <212> DNA
<400> 1
   STGACGTMR 9
<210> 2
   <211> 8
   <212> DNA
<400> 2
   STGASTMA 8
<210> 3
   <211> 8
   <212> DNA
<400> 3
   TTACCTCA 8
<210> 4
   <211> 7
   <212> DNA
<400> 4
   TCTCTTA 7
<210> 5
   <211> 12
   <212> DNA
<400> 5
   RACCACGTGGTY 12
<210> 6
   <211> 23
   <212> DNA
<400> 6
   GCTGACGTCA GACTGACGTC AGC 23
<210> 7
   <211> 24
   <212> DNA
<400> 7
   GTTTCGGGGT GACGTCACCC TTTC 24
<210> 8
   <211>24
   <212> DNA
<400> 8
   GAAAGGGACG TACGTCCGCG AAAC 24
<210> 9
   <211> 23
   <212> DNA
<400> 9
   AGATTGCCTG ACGTCAGAGA GCT 23
<210> 10
   <211> 20
   <212> DNA
<400> 10
   CGCTTGCTGA CTCAGCCGGA 20
<210> 11
   <211> 21
   <212> DNA
<400> 11
   TCAGTACTGA CTCAGTACTG A 21
<210> 12
   <211> 42
   <212> DNA
<400> 12
   ACTCTCTCAG TCTGACTCAT GCTCTCAGCA TGAGTCAGAC TG 42
<210> 13
   <211> 22
   <212> DNA
<400> 13
   TCACGGTATG ACTCATCCGT GA 22
<210> 14
   <211> 21
   <212> DNA
<400> 14
   CACGGTCTGA CTCAGACCGT G 21
<210> 15
   <211> 23
   <212> DNA
<400> 15
   TGAGTCAGTG ACTCACTGAC TCA 23
<210> 16
   <211> 21
   <212> DNA
<400> 16
   CTCCGGCTGA CTCAGCCGGA G 21
<210> 17
   <211> 23
   <212> DNA
<400> 17
   GTTACCTCAG CCCGTGAGGT AAC 23
<210> 18
   <211> 21
   <212> DNA
<400> 18
   GTTACCTCAG CCTGAGGTAA C 21
<210> 19
   <211> 22
   <212> DNA
<400> 19
   GTTACCTCAC GCGTGAGGTA AC 22
<210> 20
   <211> 20
   <212> DNA
<400> 20
   CGTTACCTCA TGAGGTAACG 20
<210> 21
   <211> 20
   <212> DNA
<400> 21
   ATTGCCTTAC CTCAGAGAGC 20
<210> 22
   <211> 26
   <212> DNA
<400> 22
   TCACCATCTC TTATGCGGTT GAATAG 26
<210> 23
   <211> 25
   <212> DNA
<400> 23
   TATGACTAAT CTCTTATTAG TCATA 25
<210> 24
   <211> 26
   <212> DNA
<400> 24
   TCTCTTAGCT CTCTTAGCCT CTCTTA 26
<210> 25
   <211> 17
   <212> DNA
<400> 25
   TAAGAGAGGC TAAGAGA 17
<210> 26
   <211> 19
   <212> DNA
<400> 26
   GAGTCTCTTA CTCCCGCGG 19
<210> 27
   <211> 17
   <212> DNA
<400> 27
   CCGCGGGTCT CTTAGAC 17
<210> 28
   <211> 24
   <212> DNA
<400> 28
   CACGGAGACC ACGTGGTCTC CGTG 24
<210> 29
   <211> 24
   <212> DNA
<401> 29
   GAAGCAGAC CACGTGGTCT GCTTC 24
<210> 30
   <211> 24
   <212> DNA
<400> 30
   AACCACGTGG TTAACCACGT GGTT 24